# EUROPÄISCHE PATENTSCHRIFT

(11) **EP 1 943 496 B1**
(45) Veröffentlichungstag und Bekanntmachung des Hinweises auf die Patenterteilung: **18.09.2013**
(21) Anmeldenummer: 06804374.4
(22) Anmeldetag: 30.10.2006
(51) Int. Cl.: G01N 17/00

(54) **KORROSIONSSENSOR**
CORROSION SENSOR
CAPTEUR DE CORROSION

(30) Priorität: 31.10.2005 AT 17812005
(43) Veröffentlichungstag der Anmeldung: 16.07.2008
(73) Patentinhaber: AC2T Research GmbH, 2700 Wiener Neustadt (AT); tecnet equity NÖ Technologiebeteiligungs-Invest GmbH, 3100 St. Pölten (AT)
(72) Erfinder: AGOSTON, Attila, A-1040 Wien (AT); JAKOBY, Bernhard, A-4040 Wien (AT)
(74) Vertreter: Margotti, Herwig Franz
(86) Internationale Anmeldenummer: PCT/AT2006/000446
(87) Internationale Veröffentlichungsnummer: WO 2007/051216

(56) Entgegenhaltungen:
- EP-A- 0 442 314
- US-A- 2 735 754
- US-A- 4 675 662
- US-A- 4 792 791
- US-A- 5 332 961
- US-B1- 6 577 140

## Beschreibung

Die Erfindung bezieht sich auf ein Sensorsystem zur Messung der Korrosivität von Flüssigkeiten, insbesondere von flüssigen Schmierstoffen (z.B. Maschinenölen).

Bei Maschinen, bei welchen z.B. zur Schmierung, Kühlung oder Kraftübertragung größere Mengen Öl verwendet werden (d. h. die Maschine hat eine bei Wartung austauschbare Ölfüllung), ist eines der Ölwechsefkriterien die Versäuerung (bzw. Korrosivität) des Öls. Das heißt, dass im Öl durch Alterung und auch durch Verschmutzung mit Fremdsubstanzen korrosiv wirkende (meist sauere) Verbindungen entstehen oder eingebracht werden, welche in der Maschine Korrosion (bzw. korrosiven Abrieb) verursachen können. Um die Maschine zu schützen, sollte das Öl gewechselt werden, bevor die Korrosion (bzw. korrosiver Abrieb) unerwünscht groß wird. Bei mit Bio-Kraftstoff betriebenen Verbrennungsmotoren ist die Korrosivität bzw. Versäuerung des Motoröls sogar das Hauptkriterium für die Durchführung eines Ölwechsels, da durch die Verunreinigungen aus den Verbrennungsgasen des oft chemisch "unreinen" Bio-Kraftstoffs viele aggressive chemische Verbindungen in das Öl gelangen, welche korrosiv sind oder durch weitere chemische Reaktionen im Öl korrosive (meist säurige) Substanzen bilden.

Für den Praxiseinsatz kommen nur wenige Methoden in Frage, um die Korrosivität bzw. Versäuerung (d. h. Säuregehalt) eines Öls zu messen. Die meisten Methoden erfordern ein entsprechend ausgerüstetes chemisches Labor, wie z. B. die Methoden zur Bestimmung der Neutralisationszahl, Bestimmung der Gesamtsäurezahl TAN (Total Acid Number) oder der Kupferstreifentest sowie der Stahlfingertest.

Eine an sich bekannte Methode zur sensorischen Messung der Korrosion von Maschinenölen ist die Verwendung eines dünnen Widerstandskupferfilms an einem isolierenden Träger, wobei die Änderung des Widerstandes des Kupferfilms infolge des Materialverlusts durch Korrosion elektrisch gemessen wird.

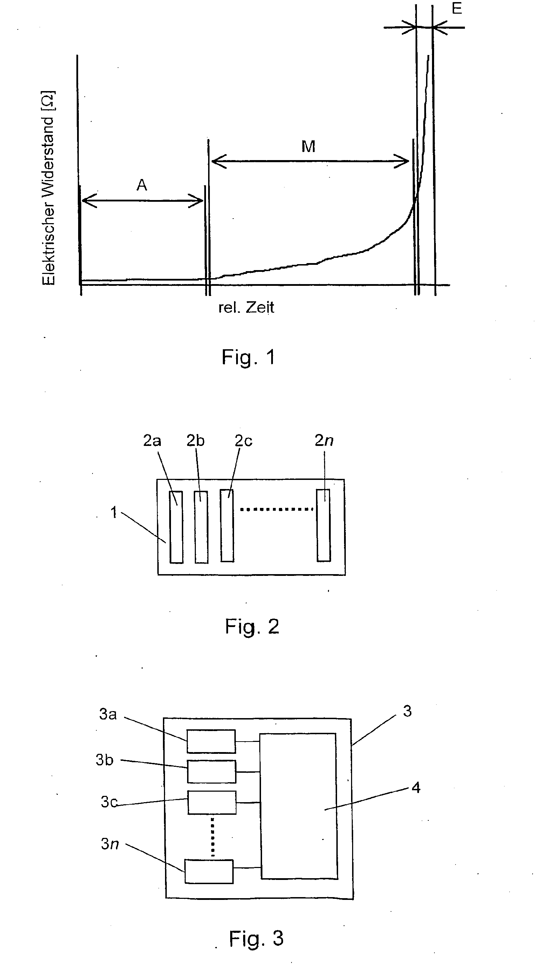

Fig. 1 zeigt ein Beispiel für den zeitlichen Verlauf der Änderung des Widerstandes eines derartigen Sensors gemäß dem Stand der Technik, während er in ein korrosives Öl eingetaucht ist. Wie in Fig. 1 erkennbar, erhöht sich der Widerstand eines derartigen Sensors quasi exponentiell. Der Nachteil dieser Methode ist, dass nur in einem zeitlich eingeschränkten Messbereich (M) die Korrosion beobachtet werden kann. Je nach Schichtdicke gibt es einen Anfangsbereich (A), wo die Widerstandsänderung noch kaum oder nur mit großem technischen Aufwand auflösbar ist, und es gibt einen Endbereich (E), wo sich der Widerstand relativ schnell über mehrere Dekaden bis zur Unterbrechung der elektrischen Verbindung erhöht. Die zuverlässige Messung des Widerstandes über mehrere Dekaden benötigt ebenfalls eine relativ aufwendige Messelektronik. Nach der Unterbrechung der elektrischen Verbindung ist keine weitere Beobachtung der Korrosionswirkung möglich.

Der Verlauf der in Fig. 1 symbolisierten Widerstandskurve hängt von der Korrosivität des Öls und von der Schichtdicke des Kupferfilms ab. Bei stärker korrosiven Ölen läuft die Korrosion des Kupferfilms und die damit verbundene Widerstandsänderung schneller, bei weniger korrosiven Ölen langsamer ab. Je dicker der Kupferlim ist, desto langsamer ändert sich dessen messbarer Widerstand während dessen Korrosion, das heißt der Anfangsbereich (A) wird länger und die Unterbrechung der elektrischen Verbindung erfolgt später. Bei dünnem Kupferfilm ändert sich der messbare Widerstand während der Korrosion schneller, das heißt der Anfangsbereich (A) wird kürzer, der Endbereich (E) wird steiler und die Unterbrechung der elektrischen Verbindung erfolgt früher.

Weiterhin ist aus der GB 1 064 485 A eine Vorrichtung und ein Verfahren zur Messung der Korrosionsraten von Metallen, die einer korrosiven Umgebung ausgesetzt sind, bekannt, wobei zumindest zwei Elektrodenpaare dieser korrosiven Umgebung ausgesetzt sind. Die Einzelelektroden jedes Elektrodenpaars sind voneinander in unterschiedlichen Abständen angeordnet, und zumindest eine Elektrode eines jeden Elektrodenpaars ist aus dem Metall gefertigt, dessen Korrosionsrate zu messen ist. An jedes Elektrodenpaar wird selektiv eine Gleichspannung angelegt, die zu einem zwischen den Elektroden gemessenen Potential von < 0,03 V führt. Weiters wird der Stromfluss zwischen Elektroden jedes Paars gemessen. Aus den Messungen der Potentiale und der Ströme wird die Korrosionsrate des Metalls bestimmt.

Die US 4,782,332 beschreibt ebenfalls ein System zur Überwachung von Schmierölen mit einem Korrosionssensor, welcher in einem Stromkreis angeordnet ist und durch Alterung des Öls korrodiert, wodurch eine Änderung des elektrischen Stromflusses durch den Sensor aufgrund der durch die Korrosion erhöhten Widerstandswerte auftritt.

Die US 6,577,140 B1 beschreibt ein weiteres System zur Überwachung von Schmierölen mit einem Korrosionssensor, wobei zwei keilförmige Elektroden, von denen zumindest eine in das Schmieröl eingetaucht wird, durch ein Dielektrikum getrennt jeweils mit einem Kapazitätsmesskreis verbunden sind. Dadurch, dass sich die zumindest eine in das Schmieröl getauchte Elektrode mit der Zeit auflöst, reduziert sich die Schichtdicke und die Fläche des durch die Elektroden gebildeten Kondensators, was zu einer Kapazitätsveränderung des Kondensators führt, die mit dem Kapazitätsmesskreis gemessen wird. Dieses bekannte System weist den Nachteil auf, dass die eine oder auch beide sich mit der Zeit auflösenden Elektroden nicht zuverlässig in den Kapazitätsmesskreis eingeschaltet werden können, da es zu Kontaktproblemen mit der sich auflösenden Elektrode kommt, die zu einem totalen Ausfall des Sensors führen können.

Aufgabe der Erfindung ist es, die eingangs beschriebenen Nachteile des Standes der Technik zu überwinden und ein Sensorsystem zur Messung der Korrosivität von Flüssigkeiten, insbesondere von flüssigen Schmierstoffen (z.B. Maschinenölen) bereitzustellen, das mit geringem apparativen Aufwand realisierbar ist, insbesondere zur Auswertung keiner Laboreinrichtung bedarf, sondern vielmehr insitu in Maschinen einbaubar und betreibbar ist und das eine hohe Auflösung der Messergebnisse bietet, wobei Kontaktprobleme und ein hierdurch möglicher Ausfall des Sensors vermieden werden soll.

Diese Aufgabe wird durch die Bereitstellung eines Sensorsystems mit den kennzeichnenden Merkmalen des Anspruchs 1 gelöst, vorteilhafte Ausgestaltungen der Erfindung sind in den Unteransprüchen dargelegt.

Das erfindungsgemäße Sensorsystem verwendet mindestens zwei flächige Elemente aus elektrisch leitfähigen Materialien (beispielsweise Metallstreifen, Folie, Metallfilm oder Blech) - im folgenden kurz "flächige Elemente" genannt -, welche flächig und/oder auch im Querschnitt (beispielsweise durch Unterschiede in der Filmdicke) strukturiert sein können und wobei mindestens eines dieser flächigen Elemente als "Korrosionsopfer" zur Erfassung der Korrosion dient, indem es mit der zu messenden korrosiven Flüssigkeit in Kontakt gehalten wird. Die gegebenenfalls strukturierten flächigen Elemente aus elektrisch leitfähigen Materialien sind in einem elektrischen Messkreis entsprechend der verwendeten Messmethode mit der Auswerteelektronik (bzw. auch miteinander) verschaltet oder verbunden. Das erfindungsgemäße Sensorsystem verwendet eine Messmethode zur Messung des korrosiven Materialverlustes des als "Korrosionsopfer" verwendeten flächigen Elementes, indem dieser über die Änderung der elektrischen Eigenschaften der als "Korrosionsopfer" verwendeten flächigen Elemente (z. B. deren elektrischer Widerstand oder Kapazität) vorzugsweise flächenhaft erfasst wird.

Dieses erfindungsgemäße Sensorsystem behebt die oben beschriebenen Nachteile des Standes der Technik und bietet einen erweiterten Messbereich und eine bessere Auflösung. Weiters erlaubt es die Implementierung einer relativ einfachen Messelektronik.

Die Vorteile der Erfindung gehen klar aus der nachfolgenden beispielhaften Erläuterung einiger Ausführungsvarianten der Erfindung anhand der vorgelegten Figuren hervor, die jedoch in keiner Weise als einschränkend zu interpretieren sind.
Fig. 1 zeigt ein Beispiel für den zeitlichen Verlauf der Änderung des Widerstandes eines Kupferfilms gemäß dem zitierten Stand der Technik, während er in ein korrosives Öl eingetaucht ist.
Fig. 2 zeigt ein Beispiel eines Sensors zur Messung der Korrosion in Ölen mit mindestens zwei flächigen Elementen.
Fig. 3 zeigt ein Beispiel für eine Mess- und Auswerteeinheit (3), welche z. B. für die in Fig. 2 dargestellte Variante des Sensorsystems verwendet werden kann.
Fig. 4 zeigt ein Beispiel für eine Messelektronik (3a, 3b, ... bis 3*n*) zur Messung des elektrischen Widerstandes der flächigen Elemente, welche in dem Beispiel gemäß Fig. 2 verwendet werden kann.
Fig. 5 zeigt beispielhaft Widerstandsmesskurven, gemessen an einem Beispiel mit vier verschieden dicken flächigen Elementen aus Kupfer, welche für die Dauer der Messung in ein bereits korrosiv wirkendes Gebrauchtöl eingetaucht wurden.
Fig. 6 zeigt ein Ausführungsbeispiel eines erfindungsgemäßen Sensoraufbaus, welcher zur Messung der Korrosion in Ölen mit der erfindungsgemäßen Messmethode verwendet werden kann.
Fig. 7 zeigt schematisch ein Ausführungsbeispiel der chemisch stabilen flächigen Elemente (Messelektroden) (6) zur kapazitiven Messung der elektrischen Eigenschaft eines als "Korrosionsopfer" verwendeten flächigen Elementes zur Messung des korrosiven Materialverlustes.
Fig. 8 zeigt ein Ausführungsbeispiel, bei dem die chemisch stabilen flächigen Elemente (6) als kapazitive Messelektroden auf demselben Träger (7) gegenüber den zu korrodierenden flächigen Elementen (2) auf der Rückseite des Trägers (7) aufgebracht sind.
Fig. 9 zeigt eine Ausführungsvariante, wo die sich durch Korrosion ändernde elektrische Eigenschaft des als "Korrosionsopfer" verwendeten, gegebenenfalls strukturierten flächigen Elementes bzw. der flächigen Elemente (2) induktiv erfasst wird.

Fig. 2 zeigt ein Beispiel eines Sensors zur Messung der Korrosion in Ölen mit mindestens zwei flächigen Elementen. Die flächigen Elemente dienen hier als "Korrosionsopfer" (2a), (2b), (2c) ... bis (2*n*) mit unterschiedlichen Filmdicken und sind auf einem nicht leitenden ölbeständigen Träger (1) aufgebracht. Die flächigen Elemente (2a), (2b), (2c) ... bis (2*n*) sollen aus einem Metall sein, welches für die in der jeweiligen Anwendung auftretende Korrosivität des Öles anfällig ist und/oder in der für die Korrosion kritischen Teilen der Maschine verwendet wird. Durch Verwendung einer beliebigen Anzahl *n* von flächigen Elementen (2a), (2b), (2c) ... bis (2*n*) ist eine beliebig feine Abstufung und Erweiterung des Messbereiches für die Korrosion möglich. Die flächigen Elemente (2a), (2b), (2c) ... bis (2*n*) sind in diesem Falle vorzugsweise als Widerstandsbahnen ausgeführt, welche an zwei Enden elektrisch leitend kontaktiert und mit den zugeordneten Messelektronik-Komponenten (3a), (3b), (3c) ... bis (3*n*) der Mess- und Auswerteeinheit (3) zu einem elektrischen Messkreis (siehe Fig. 3) verbunden sind.

Es sei erwähnt, dass die flächigen Elemente nicht auf ebene Flächen eingeschränkt sind, sondern eine beliebige Form im Raum einnehmen können, die z.B. durch Ausschneiden und Biegen herstellbar ist.

Ein Beispiel für eine Mess- und Auswerteeinheit (3), welche z. B. für die in Fig. 2 dargestellten Variante des Sensorsystems verwendet werden kann, zeigt Fig. 3. Die Mess- und Auswerteeinheit (3) hat in diesem Falle für jedes der flächigen Elemente (2a), (2b), (2c) ... bis (2*n*) eine Messelektronik (3a), (3b), (3c) ... bis (3*n*) zur Messung des elektrischen Widerstandes der flächigen Elemente. Die Ausgangssignale der Messelektroniken (3a), (3b), (3c) ... bis (3*n*) werden zur Auswerteeinheit (4) weitergeleitet, wo diese weiter verarbeitet und als Messwert ausgegeben bzw. - z. B auf einer Anzeigeeinheit (Display) - dargestellt werden können.

Durch die Verwendung von mehr als einem flächigen Element mit unterschiedlicher Filmdicke in dieser Ausführungsvariante ist es ausreichend, die relative Widerstandsänderung nur in einem kleineren, für die Messelektronik technisch einfach realisierbaren Bereich zu messen, vorzugsweise nur über zwei oder weniger Dekaden. Durch Verwendung mehrerer flächiger Elemente mit unterschiedlicher Filmdicke ist auch eine digitale Auslesung (Erkennung des Zustandes "leitend" bzw. "nicht-leitend") des Sensors möglich, wobei die Auflösung von der Anzahl n der flächigen Elemente bestimmt wird.

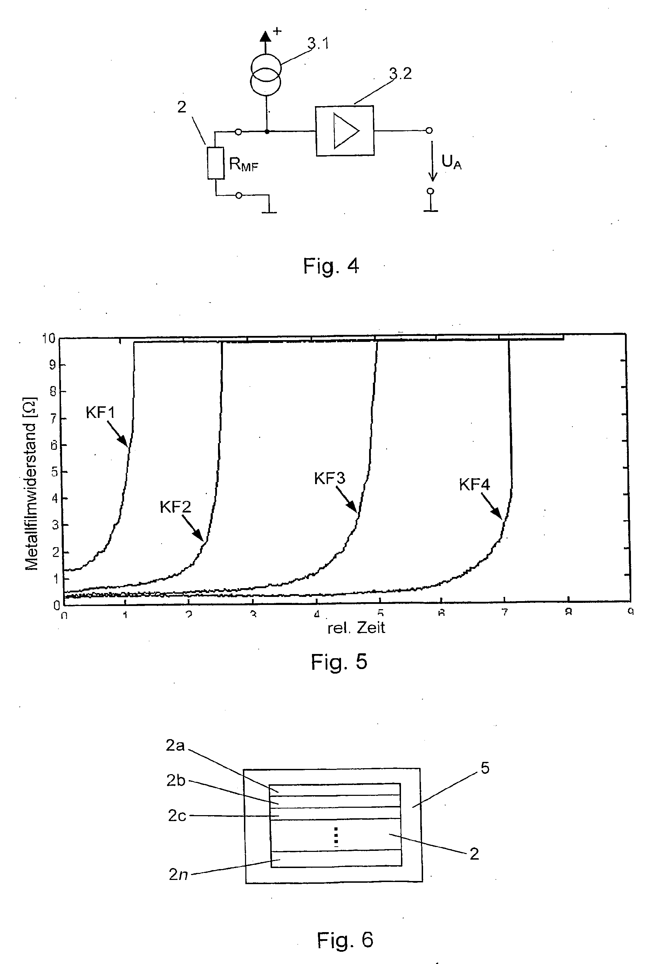

Fig. 4 zeigt ein Beispiel für eine Messelektronik (3) zur Messung des elektrischen Widerstandes der flächigen Elemente, welche in dem Ausführungsbeispiel gemäß Fig. 2 verwendet werden kann. Durch den Widerstand R_{MF} zwischen den elektrisch leitenden Kontaktierungen an zwei Enden eines zu messenden flächigen Elementes (2) wird ein durch die Stromquelle (3.1) definierter Strom geleitet. Ein Spannungsverstärker (3.2) kann verwendet werden, um das Spannungsabfallsignal an dem Widerstand des flächigen Elementes für die Weiterverarbeitung zu verstärken.

Wenn der Sensor in eine Maschine so eingebaut wird, dass der Sensor ständig ins Öl eintaucht, d. h. mit dem Öl in Kontakt ist, erfahren die flächigen Elemente auf dem Sensor denselben Korrosionsangriff des Öls während der Betriebs- und auch Stillstandzeiten der Maschine, wie die metallischen Teile der Maschine selbst, d. h. die durch die flächigen Elemente des Sensors als "Korrosionsopfer" erfahrene Korrosion repräsentiert die gesamte Korrosionswirkung, welche auch die metallischen Teile der Maschine selbst erfahren. Die so aufintegrierte Gesamtkorrosionswirkung kann bereits als Maß für ein Ölwechselkriterium dienen.

Da die Korrosivität (bzw. der Säuregehalt) des Öles im Laufe des Betriebs zunimmt, könnte es vorteilhaft sein, die Korrosivität bzw. Korrosionswirkung des Öles in einem bestimmten Zeitpunkt bzw. einer bestimmten Zeitspanne zu bestimmen. Die Korrosivität des Öles bestimmt, wie schnell die Korrosionskurve (wie in Fig. 1 symbolisiert) für eine bestimmte Filmdicke durchlaufen wird. Durch Vergleich zu einem Referenzverlauf, welcher mit Hilfe eines konstantkorrosiven Öles bestimmt wird, ist es möglich, Korrosivität bzw. Korrosionswirkung des Öles, bei welchem sich die Korrosivität mit der Zeit ändert, in einem bestimmten Zeitpunkt bzw. Zeitspanne zu bestimmen. Durch Verwendung von mehr als einem flächigen Element aus elektrisch leitfähigen Materialien erfolgt eine feinere Abstufung, d. h. Auflösung, des Messbereiches, wodurch die Messgenauigkeit wesentlich verbessert wird.

Es sei erwähnt, dass die sich auf eine Messung des elektrischen Widerstandes des als "Korrosionsopfer" verwendeten flächigen Elements durch direktes Einschalten in den Messkreis beziehenden Beispiele nicht Teil der Erfindung und nur zum besseren Verständnis der Erfindung angeführt wurde.

Fig 5 zeigt beispielhaft Widerstandsmesskurven gemessen an einem Beispiel mit vier verschieden dicken flächigen Elementen aus Kupfer, welche für die Dauer der Messung in ein bereits korrosiv wirkendes Gebrauchtöl eingetaucht wurden. Bei diesem Ausführungsbeispiel sind die flächigen Elemente als Kupferfilm ausgebildet und 100 nm (KF1), 300 nm (KF2), 600 nm (KF3) und 1000 nm (KF4) dick. Es ist erkennbar, dass durch die geeignete Abstufung der Filmdicken eine Überlappung der Messbereiche erreicht werden kann. In diesem Beispiel kann das Voranschreiten der Korrosion sowohl bei noch geringer als auch bei großer Gesamtkorrosion noch aufgelöst werden, ohne dass der Widerstand über mehrere Dekaden gemessen werden muss.

Die Korrosion eines Metalls erfolgt im allgemeinen nicht gleichmäßig über die ganze Fläche, sondern fleckig, ausgehend von so genannten Korrosionskelmen (Korrosionszentren). Dies ist mitunter der Grund, dass der Widerstand eines hinreichend dünnen flächigen Elementes (Metallfilmes) während der Korrosion sprungartig zunehmen kann. In einem ungünstigen Fall kann es vorkommen, dass der elektrische Kontakt an einer Stelle des flächigen Elementes durch die Korrosion bereits unterbrochen ist, obwohl der Großteil der Metallfilmfläche noch vorhanden ist. Deshalb ist die Messung des elektrischen Widerstandes eines flächigen Elementes nur bedingt bzw. nicht in allen Fällen geeignet, um den korrosiven Materialverlust, etwa eines Metallfilms, zu messen.

Die vorgeschlagene erfindungsgemäße Messmethode gestattet mit einem hierzu ebenfalls vorgeschlagenen Sensoraufbau, die oben genannten Einschränkungen zu beheben und eine messtechnische Beobachtung der gesamten zur Erfassung der Korrosion eingesetzten flächigen Elemente als "Korrosionsopfer" zu ermöglichen.

Der erfindungsgemäße Sensoraufbau zur Messung der Korrosion in Ölen mit der erfindungsgemäßen Meßmethode sieht mindestens ein flächiges Element vor, welches als "Korrosionsopfer" (Opferschicht) zur Erfassung der Korrosion dient. Fig. 6 zeigt ein Ausführungsbeispiel eines erfindungsgemäßen Sensoraufbaus. Mindestens ein flächiges Element aus elektrisch leitfähigem Material (2) ist auf einem nicht leitenden ölbeständigen Träger (5) aufgebracht. Dabei gelten die im ersten Teil der Erfindung beschriebenen erfindungsgemäßen Grundsätze auch hier. Das heißt, durch Verwendung mehrerer flächiger Elemente aus elektrisch leitfähigen Materialien unterschiedlicher Filmdicke ist auch bei diesem Sensoraufbau eine weitere Verfeinerung und Erweiterung des Messbereiches für die Korrosion möglich. Die im Weiteren beschriebene Variante der erfindungsgemäßen Messmethode erlaubt auch, dass die flächigen Elemente unterschiedlicher Filmdicke sich elektrisch leitend berühren, d. h. dass das als "Korrosionsopfer" verwendete flächige Element im Querschnitt (in der Filmdicke) strukturiert ist.

Die erfindungsgemäße Messmethode zur Messung des korrosiven Materialverlustes mit wenigstens einem als "Korrosionsopfer" verwendeten flächigen Element sieht in einer anderen Ausführungsvariante die kapazitive Messung der elektrischen Eigenschaft der flächigen Elemente vor. Hierbei kann durch eine flächige Strukturierung der chemisch stabilen flächigen Elemente im Sinne von Masselektroden, vorzugsweise durch eine an sich bekannte inter-digitale (d.h. kammartig ineinander greifende) Struktur, im wesentlichen parallel zum Flächenverlauf des als "Korrosionsopfer" verwendeten flächigen Elementes bzw. der als "Korrosionsopfer" verwendeten flächigen Elemente die Summe der lokalen Flächenleitfähigkeiten gemessen werden.

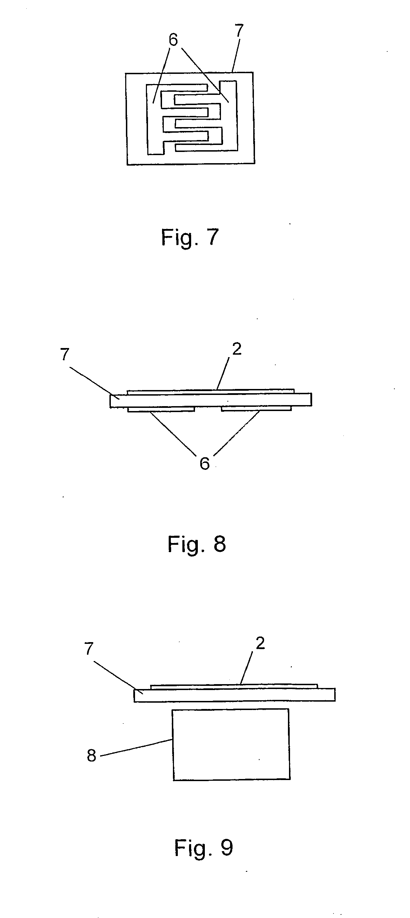

Fig. 7 zeigt schematisch ein Ausführungsbeispiel der chemisch stabilen flächigen Elemente (6), die als Messelektroden fungieren, zur kapazitiven Messung der elektrischen Eigenschaft eines als "Korrosionsopfer" verwendeten flächigen Elementes zur Messung des korrosiven Materialverlustes. Das "Korrosionsopfer" liegt parallel zu den chemisch stabilen flächigen Elementen (6) an der Rückseite des Trägers (7) und ist daher in Fig. 7 nicht zu sehen. Die chemisch stabilen flächigen Elemente (6) sind aus einem elektrisch leitfähigen, korrosionsbeständigen Material oder mit an sich bekannten Beschichtungen bzw. Methoden vor Korrosion geschützt. Die chemisch stabilen flächigen Elemente (6) sind in diesem Beispiel auf einem Träger (7) aus einem elektrisch isolierenden ölbeständigen Material aufgebracht, können alternativ dazu auch in eine Halterung aus solchem Material montiert sein. Es ist auch eine Ausführungsvariante vorgesehen, bei der die chemisch stabilen flächigen Elemente (6) und die als "Korrosionsopfer" eingesetzten flächigen Elemente (2) an gegenüberliegenden Oberflächen eines auswechselbaren Trägers oder einer auswechselbaren Halterung angeordnet sind, so dass sie rasch und mit geringem Arbeitsaufwand ausgetauscht werden können.

Die flächigen Elemente (2, 6) und gegebenenfalls der Träger oder die Halterung können auch flexibel ausgeführt sein, was eine Formanpassung durch Ausschneiden und Biegen erlaubt. Der sich daraus ergebende Vorteil ist z.B., dass der so ausgebildete Korrosionssensor auch als ein Teilstück einer Rohrwand ausgeführt sein bzw. so in einem Rohr untergebracht werden kann, dass die Strömung einer Flüssigkeit im Rohr nicht wesentlich beeinflusst wird. Diese ist auch gegeben, wenn die flächigen Elemente zwar starr sind, aber herstellungstechnisch in die gewünschte Form gebracht werden.

Fig. 8 zeigt ein Ausführungsbeispiel, bei dem die chemisch stabilen flächigen Elemente (6) als kapazitive Messelektroden auf demselben Träger (7) gegenüber den zu korrodierenden flächigen Elementen (2) ("Korrosionsopfer") aufgebracht sind, welche auf der Rückseite des Trägers (7) aufgebracht sind. Mit dieser Anordnung ist es möglich, zum Beispiel durch Anlegen einer Wechselspannung an die chemisch stabilen flächigen Elemente (6), die Kopplungskapazitäten zwischen den chemisch stabilen flächigen Elementen (6) und dem "Korrosionsopfer" (2) sowie den verteilten elektrischen Widerstand des "Korrosionsopfers" (2) zu bestimmen. Die Kopplungskapazität ist im Fall einer fleckigen, Loch bildenden Korrosion ein Maß für die verbleibende Fläche des "Kon-osionsopfers" (2). Bei der Bestimmung der verbleibenden Fläche bzw. des verteilten Widerstands ist die Funktion des chemisch stabilen flächigen Elementes (6) die großflächige und flächenhafte elektrische Ankopplung an das Korrosionsopfer (2) bzw. dass es eine kontaktlose Messung erlaubt und dadurch die Wartung vereinfacht.

Fig. 9 zeigt eine Ausführungsvariante, bei der die sich durch Korrosion ändernde elektrische Eigenschaft des als "Korrosionsopfer" verwendeten, gegebenenfalls strukturierten flächigen Elementes bzw. der flächigen Elemente (2) induktiv mit Hilfe einer elektrischen Spule (8) erfasst wird, welche auf der Rückseite des Trägers (7) durch entsprechende Strukturierung eines flächigen Elementes (etwa eines Metallfilmes) als Spule ausgebildet sein kann.

Es sei betont, dass die Verwendung mehrerer verschiedener Dickenschichten des als "Korrosionsopfer" verwendeten flächigen Elements (2) die Messtechnik vereinfacht und den Messbereich erweitert. Weiters wird durch die Verwendung eines flächigen Messverfahrens sowohl gleichmäßiger als auch fleckiger Korrosions-Materialverlust erfasst und dadurch zusätzliche Information über den Korrosionsmechanismus gewonnen. Außerdem muss das "Korrosionsopfer" nicht elektrisch leitend kontaktiert werden, wodurch das Problem beseitigt wird, dass Korrosion bevorzugt an Kontaktstellen stattfindet. Ein weiterer Vorteil dieser Ausgestaltung ist, dass eine einfacherer Wartung ermöglicht wird, da dadurch das Korrosionsopfer leicht tauschbar ist. Denn bei elektrisch kontaktierender Messung muss wegen Kontaktierungsproblemen die ganze Einheit getauscht werden, welche in das Öl hinein ragt.

Im Falle der induktiven Ankopplung ist der induzierte Wirbelstrom bzw. die dadurch verursachte Dämpfung ein Maß für den verteilten elektrischen Widerstand des Korrosionsopfers, wobei dies vom leitenden Volumen der Opferschicht abhängt, egal ob Material gleichmäßig oder in Flecken verteilt ist.

Im Fall kapazitiver Ankopplung ist bei entsprechender Anordnung der Messelektroden sowohl der Flächenanteil als auch der verteilte Widerstand bestimmbar.

## Patentansprüche

1. Sensorsystem zur Messung der Korrosivität von Flüssigkeiten, insbesondere von flüssigen Schmierstoffen (Maschinenölen), wobei mindestens zwei flächige Elemente (2 bzw. 6) aus elektrisch leitfähigen Materialien in einem elektrischen Messkreis vorgesehen sind, wobei mindestens eines dieser flächigen Elemente aus einem Material besteht, welches für die Korrosionswirkung der betreffenden Flüssigkeit anfällig ist und als "Korrosionsopfer" (2) mit der zu messenden Flüssigkeit in Kontakt bringbar ist, **dadurch gekennzeichnet, dass** der elektrische Messkreis eine durch den korrosiven Materialverlust des zumindest einen als "Korrosionsopfer" verwendeten flächigen Elementes (2) hervorgerufene Änderung von mindestens einer elektrischen Wechselspannungs-Koppelungseigenschaft zwischen den zumindest zwei flächigen Elementen (2, 6) erfasst, wobei der elektrische Messkreis die mindestens eine durch den korrosionsbedingten Materialverlust des als "Korrosionsopfer" verwendeten flächigen Elementes (2) veränderte elektrische Wechselspannungs-Koppelungseigenschaft zwischen den zumindest zwei flächigen Elementen (2, 6) kontaktlos kapazitiv und/oder kontaktlos induktiv gekoppelt erfasst.

2. Sensorsystem nach Anspruch 1, **dadurch gekennzeichnet, dass** die flächigen Elemente (2) flächig und/oder im Querschnitt strukturiert sind.

3. Sensorsystem nach Anspruch 1 oder 2, **dadurch gekennzeichnet, dass** zumindest zwei als "Korrosionsopfer" vorgesehene flächige Elemente (2a ... 2n) unterschiedlicher Dicke vorgesehen sind.

4. Sensorsystem nach einem der Ansprüche 1 bis 3, **dadurch gekennzeichnet, dass** zumindest eines der elektrisch leitfähigen, flächigen Elemente (6) chemisch stabil, d.h. gegen Korrosion beständig, ausgeführt ist.

5. Sensorsystem nach einem der Ansprüche 1 bis 4, **dadurch gekennzeichnet, dass** ein als "Korrosionsopfer" verwendetes flächiges Element (2) über zumindest ein chemisch stabiles, elektrisch leitfähiges, flächiges Element (6) an den Messkreis (3) ankoppelbar ist.

6. Sensorsystem nach Anspruch 5, **dadurch gekennzeichnet, dass** chemisch stabile flächige Elemente (6) eine flächige Strukturierung aufweisen, vorzugsweise eine inter-digitale bzw. kammartige Struktur, und im wesentlichen parallel zum Flächenverlauf des als "Korrosionsopfer" verwendeten flächigen Elementes (2) bzw. der als "Korrosionsopfer" verwendeten flächigen Elemente angeordnet sind, wobei die chemisch stabilen flächigen Elemente (6) an den Messkreis angeschlossen sind.

7. Sensorsystem nach einem der Ansprüche 1 bis 6, **dadurch gekennzeichnet, dass** die elektrisch leitfähigen, flächigen Elemente (2, 6) auf mindestens einem Träger aus einem elektrisch isolierenden, gegenüber der Flüssigkeit beständigen Material (5 bzw. 7) aufgebracht oder in mindestens eine Halterung aus solchem Material montiert sind.

8. Sensorsystem nach einem der Ansprüche 4 bis 7, **dadurch gekennzeichnet, dass** die chemisch stabilen, elektrisch leitfähigen, flächigen Elemente (6) den als "Korrosionsopfer" verwendeten flächigen Elementen (2) gegenüberliegend angeordnet sind, wobei sie optional auf einem gemeinsamen Träger (7), insbesondere auf gegenüberliegenden Seiten des Trägers (7), aufgebracht oder in einer gemeinsamen Halterung untergebracht sind.

9. Sensorsystem nach einem der Ansprüche 1 bis 8, **dadurch gekennzeichnet, dass** die als "Korrosionsopfer" verwendeten flächigen Elemente (2) auf einem austauschbaren Träger (5) aufgebracht oder austauschbar in einer Halterung untergebracht sind.

10. Sensorsystem nach einem der vorhergehenden Ansprüche, **dadurch gekennzeichnet, dass** die flächigen Elemente (2a ... 2n) einander elektrisch leitend berühren.

11. Sensorsystem nach einem der vorhergehenden Ansprüche, **dadurch gekennzeichnet, dass** eine sich durch den korrosiven Materialverlust des als "Korrosionsopfer" verwendeten flächigen Elementes (2) ändernde elektrische Wechselspannungs-Koppelungseigenschaft die elektrische Kapazität zwischen zumindest zwei flächigen Elementen (2, 6) ist.

12. Sensorsystem nach einem der vorhergehenden Ansprüche, **dadurch gekennzeichnet, dass** eine Spule (8) oder ein als Spule (Spirale) ausgebildetes flächiges Element (6) vorgesehen ist, die/das induktiv an zumindest ein flächiges, vorzugsweise als "Korrosionsopfer" verwendetes Element (2, 6) gekoppelt ist.

13. Sensorsystem nach einem der vorhergehenden Ansprüche, **dadurch gekennzeichnet, dass** eine sich durch den korrosiven Materialverlust des als "Korrosionsopfer" verwendeten flächigen Elementes (2) ändernde elektrische Wechselspannungs-Koppelungseigenschaft der verteilte durchschnittliche Widerstand ist, wobei chemisch stabile flächige Elemente (6) mit einer flächigen Ankoppelung zum als "Korrosionsopfer" verwendeten flächigen Element (2) ausgeführt sind.

## Claims

1. A sensor system for measuring the corrosiveness of liquids, in particular liquid lubricants (machine oils), wherein at least two planar elements (2 and 6, respectively) made of electrically conductive materials are provided in an electrical measuring circuit, with at least one of said planar elements consisting of a material which is susceptible to the corrosion effect of the respective liquid and, as a "corrosion victim" (2), can be brought into contact with the liquid to be measured, **characterized in that** the electrical measuring circuit detects a change in at least one electrical alternating-voltage coupling property between the at least two planar elements (2, 6), which has been caused by the corrosive loss of material of the at least one planar element (2) used as a "corrosion victim", wherein the electrical measuring circuit detects the at least one electrical alternating-voltage coupling property between the at least two planar elements (2, 6), which property has changed due to the corrosion-induced loss of material of the planar element (2) used as a "corrosion victim", in a contactless capacitively and/or contactless inductively coupled manner.

2. A sensor system according to claim 1, **characterized in that** the planar elements (2) are planar and/or structured in their cross-sections.

3. A sensor system according to claim 1 or 2, **characterized in that** at least two planar elements (2a ... 2n) provided as "corrosion victims" and having different thicknesses are provided.

4. A sensor system according to any of claims 1 to 3, **characterized in that** at least one of the electrically conductive planar elements (6) is designed so as to be chemically stable, i.e., resistant against corrosion.

5. A sensor system according to any of claims 1 to 4, **characterized in that** a planar element (2) used as a "corrosion victim" can be coupled to the measuring circuit (3) via at least one chemically stable, electrically conductive planar element (6).

6. A sensor system according to claim 5, **characterized in that** chemically stable planar elements (6) have a planar structuring, preferably an interdigital or, respectively, comb-like structure, and are arranged essentially parallel to the surface progression of the planar element (2) used as a "corrosion victim" or, respectively, of the planar elements used as "corrosion victims", with the chemically stable planar elements (6) being connected to the measuring circuit.

7. A sensor system according to any of claims 1 to 6, **characterized in that** the electrically conductive planar elements (2, 6) are applied on at least one carrier made of an electrically insulating material (5 and 7, respectively) which is resistant against the liquid or are mounted in at least one fixing device made of such a material.

8. A sensor system according to any of claims 4 to 7, **characterized in that** the chemically stable, electrically conductive planar elements (6) are arranged opposite the planar elements (2) used as "corrosion victims", wherein, optionally, they are applied on a common carrier (7), in particular at opposite sides of the carrier (7), or are accommodated in a common fixing device.

9. A sensor system according to any of claims 1 to 8, **characterized in that** the planar elements (2) used as "corrosion victims" are applied on an exchangeable carrier (5) or are accommodated in a fixing device in a replaceable manner.

10. A sensor system according to any of the preceding claims, **characterized in that** the planar elements (2a ... 2n) contact each other in an electrically conducting manner.

11. A sensor system according to any of the preceding claims, **characterized in that** an electrical alternating-voltage coupling property changing due to the corrosive loss of material of the planar element (2) used as a "corrosion victim" is the electrical capacitance between at least two planar elements (2, 6).

12. A sensor system according to any of the preceding claims, **characterized in that** a coil (8) or a planar element (6) formed as a coil (helix) is provided which is coupled inductively to at least one planar element (2, 6) preferably used as a "corrosion victim".

13. A sensor system according to any of the preceding claims, **characterized in that** an electrical alternating-voltage coupling property changing due to the corrosive loss of material of the planar element (2) used as a "corrosion victim" is the distributed mean resistance, wherein chemically stable planar elements (6) are designed with a planar coupling to the planar element (2) used as a "corrosion victim".

## Revendications

1. Système de capteur pour la mesure de la corrosivité de liquides, en particulier de produits lubrifiants liquides (huiles de lubrification de machines), au moins deux éléments plans (2, respectivement 6) constitués de matériaux électriquement conducteurs étant prévus dans un circuit de mesures électriques, au moins l'un de ces éléments plans étant constitué d'un matériau qui est sensible à l'influence de la corrosion par le liquide en question et qui peut être mis en contact en tant que « élément de corrosion sacrificiel » (2) avec le liquide à évaluer, **caractérisé en ce que** le circuit électrique capte une variation d'au moins une propriété du couplage de tension électrique alternative au moins entre les deux éléments plans (2, 6) induite par au moins un des éléments plans (2) employés en tant « qu'élément de corrosion sacrificiel », le circuit de mesure électrique captant au moins une propriété de couplage de tension électrique alternative modifiée par la perte de matière induite par la corrosion de l'élément plan (2) utilisé comme « élément de corrosion sacrificiel » entre au moins les deux éléments plans (2, 6) couplés par les capacités sans contact et/ou par induction sans contact.

2. Système de capteur selon la revendication 1 **caractérisé en ce que** les éléments plans (2) sont plans et/ou structurés dans leur section transversale.

3. Système selon les revendications 1 ou 2 **caractérisé en ce qu'**au moins deux éléments plans (2a ... 2n) prévus en tant « qu'éléments de corrosion sacrificiels » sont prévus à des épaisseurs différentes.

4. Système de capteur selon l'une des revendications de 1 à 3 **caractérisé en ce qu'**au moins un des éléments plans (6) pouvant être électriquement conducteur est conçu chimiquement stable, c'est-à-dire résistant à la corrosion.

5. Système de capteur selon l'une des revendications de 1 à 4 **caractérisé en ce qu'**un élément plan (2) utilisé en tant « qu'élément de corrosion sacrificiel » peut être couplé au circuit de mesure (3) par l'intermédiaire d'au moins un élément plan (6) chimiquement stable, électriquement conducteur.

6. Système de capteur selon la revendication 5 **caractérisé en ce que** des éléments plans (6) chimiquement stables présentent une structuration plane, de préférence une structure interdigitale, respectivement sous la forme d'un peigne, et qu'ils sont disposés dans l'ensemble parallèles par rapport au plan de l'élément plan (2) utilisé en tant « qu'élément de corrosion sacrificiel », respectivement par rapport à l'élément plan utilisé en tant « qu'élément de corrosion sacrificiel », les éléments plans (6) chimiquement stables étant connectés au circuit de mesure.

7. Système de capteur selon l'une des revendications de 1 à 6 **caractérisé en ce que** des éléments plans (2, 6) électriquement conducteurs sont rapportés sur au moins un support constitué d'un matériau (5, respectivement 7) électriquement isolant, résistant par rapport au liquide ou sont au moins montés sur un système de maintien constitué d'un tel matériau.

8. Système de capteur selon l'une des revendications de 4 à 7 **caractérisé en ce que** les éléments plans (6) chimiquement stables, électriquement conducteurs sont disposés en face des éléments plans (2) utilisés en tant « qu'éléments de corrosion sacrificiels », pouvant être éventuellement rapportés sur un support (7) commun, en particulier sur des côtés du support (7) se faisant face, ou pouvant être logés dans un équipement de maintien commun.

9. Système selon l'une des revendications de 1 à 8 **caractérisé en ce que** les éléments plans (2) utilisés en tant « qu'éléments de corrosion sacrificiels » sont rapportés sur un support (5) échangeable ou peuvent être logés de manière échangeable dans un équipement de maintien.

10. Système de capteur selon l'une des revendications précédentes **caractérisé en ce que** les éléments plans (2a ... 2n) sont en contact électriquement conducteur.

11. Système de capteur selon l'une des revendications précédentes **caractérisé en ce que** la caractéristique du couplage de la tension électrique alternative se modifiant par la perte de matière par corrosion de l'élément plan (2) utilisé comme « élément de corrosion sacrificiel » constitue la capacité électrique entre au moins deux éléments plans (2, 6).

12. Système de capteur selon l'une des revendications précédentes **caractérisé en ce qu'**une bobine ou qu'un élément plan (6) conçu en tant que bobine (spirale) est prévu, qui est couplé de manière inductive à au moins un élément (2, 6), plan, utilisé de préférence en tant « qu'élément de corrosion sacrificiel ».

13. Système de capteur selon l'une des revendications précédentes **caractérisé en ce qu'**une caractéristique du couplage de la tension électrique alternative se modifiant par la perte de matière par corrosion de l'élément plan (2) utilisé en tant « qu'élément de corrosion sacrificiel » est la résistance moyenne distribuée, des éléments plans 6) chimiquement stables étant organisés avec un couplage avec l'élément plan (2) utilisé comme « élément de corrosion sacrificiel ».
